(19)

**Europäisches Patentamt**
**European Patent Office**
**Office européen des brevets**

(11)     **EP 1 542 742 B1**

(12)     **EUROPEAN PATENT SPECIFICATION**

(45) Date of publication and mention
of the grant of the patent:
**31.03.2010  Bulletin 2010/13**

(51) Int Cl.:
***A61M 5/00*** *(2006.01)*

(21) Application number: **03798659.3**

(22) Date of filing: **07.04.2003**

(86) International application number:
**PCT/US2003/010713**

(87) International publication number:
**WO 2004/028595 (08.04.2004 Gazette 2004/15)**

(54) **ENTERAL FEEDING PUMP HAVING A SAFETY INTERLOCK SYSTEM**

ENTERALE ZUFÜHRPUMPE MIT SICHERHEITS-VERRIEGELUNGSSYSTEM

POMPE D'ALIMENTATION ENTERALE AVEC SYSTEME DE VERROUILLAGE DE SECURITE

(84) Designated Contracting States:
**AT BE BG CH CY CZ DE DK EE ES FI FR GB GR HU IE IT LI LU MC NL PT RO SE SI SK TR**

(30) Priority: **26.09.2002  US 413710 P**

(43) Date of publication of application:
**22.06.2005  Bulletin 2005/25**

(73) Proprietor: **Covidien AG**
**8212 Neuhausen am Rheinfall (CH)**

(72) Inventors:
• **PRICE, Jeffrey**
**Wildwood, MO 63040 (US)**
• **FOURNIE, Glenn, G.**
**Smithton, IL 62285 (US)**

• **HUDSON, Joseph, A.**
**O'Fallon, MO 63366 (US)**
• **SISK, Ricky, A.**
**Washington, MO 63090 (US)**
• **NAUMANN, Richard, J.**
**Chesterfield, MO 63017 (US)**

(74) Representative: **Tomlinson, Edward James**
**Frohwitter**
**Patent- und Rechtsanwälte**
**P.O. Box 86 03 68**
**81630 München (DE)**

(56) References cited:
**US-A- 4 563 179     US-A- 4 884 013**
**US-A- 5 201 711     US-A- 5 462 525**

## Description

BACKGROUND OF THE INVENTION

1. <u>Field of the Invention</u>

The present invention relates to a safety interlock system, and more particularly to a safety interlock system for a flow control apparatus. More specifically, the present invention relates to a safety interlock system using a magnet-less administration feeding set with an enteral feeding pump.

2. <u>Prior Art</u>

Enteral feeding systems are frequently used for patients who are unable to take nutrition alone and require some type of feeding system to provide nutrition through a gastrointestinal tract of a patient, such as the stomach. Typically, an enteral feeding system comprises a disposable administration feeding set in conjunction with a flow control apparatus, such as a pump, for supplying fluid to a patient at a controlled delivery rate. The administration feeding set of the prior art includes an inlet tube in communication with a source of fluid at one end and a drip chamber which is arranged to be mounted along a first recess on the pump at the other end, a mounting member for mounting the administration feeding set to a second recess on the pump, and a pump tube which connects the drip chamber to the mounting member while engaging a motor driven rotor on the pump.

In many enteral feeding systems the engagement of the pump tube to the rotor controls the flow of fluid to the patient according to the speed of the rotor. In the event the administration feeding set is not properly mounted to the pump, an excess flow of fluid through the feeding set can occur under force of gravity which is highly undesirable. Improper mounting of the drip chamber is unlikely because of the mechanical configuration of that component and its corresponding recesses on the pump. However, improper placement of the mounting member, e.g. below, above or outside of the respective recess on the pump is more likely if the administration feeding set is improperly installed on the pump by an inexperienced operator who has not yet received proper instruction in the operation of the enteral feeding system. Instances of such improper installation of the administration feeding set have been reported.

U.S. Patent Nos. 4,913,703; 5,201,711; and 6,017,326 to Pasqualucci et al. disclose a safety interlock system for a flow control apparatus provided with a magnetic field source in the region of its mounting to an administration feeding set having a magnetic source, such as a magnet, incorporated into the mounting member. The flow control apparatus includes a magnetic field sensitive switching component which detects the proper placement of administration feeding set in the recess of the flow control apparatus and prevents operation of the flow control apparatus unless the mounting member of the administration feeding set is in a properly engaged position to the pump. Although the safety interlock system of Pasqualucci et al. provides an excellent means of ensuring proper engagement of the administration feeding set on the flow control apparatus, the magnet used to provide the magnetic source on the disposable administration feeding set makes it relatively expensive to manufacture.

Therefore, there appears a need in the art for a safety interlock system that is inexpensive to manufacture. There is a further need in the art for a safety interlock system that does not require an administration feeding set having a magnet in order to ensure proper placement of the feeding set on the flow control apparatus.

OBJECTS AND SUMMARY OF THE INVENTION

The invention is defined in claim 1. It includes an enteral feeding system that ensures the proper placement of the administration feeding set on a flow control apparatus with the administration feeding set having no magnetic source, such as a magnet.

A such administration feeding set is inexpensive to manufacture.

Moreover, the present invention is to provide a safety interlock system having a sensor arrangement inside the flow control apparatus for detection of a magnetically-susceptible component on the administration feeding set.

The sensor arrangement inside the flow control apparatus senses a variation in the magnetic field when the feeding set is properly installed.

Preferred embodiment of the present invention are defined in the dependent claims.

In brief summary, the present invention overcomes and substantially alleviates the deficiencies in the prior art by comprising a safety interlock system for an enteral feeding system comprising a magnet-less administration feeding set adapted for operative engagement with a flow control apparatus for delivery of fluid at a controlled rate to a patient.

There is also presented a method for preventing improper system operation which comprises the steps of providing a sensor arrangement inside the flow control apparatus which is operatively associated with a switching component that places the flow control apparatus in a first electrical state when the administration feeding set is properly mounted to the flow control apparatus and a second electrical state when the administration feeding set is not properly mounted to the flow control apparatus. In operation, the flow control apparatus is enabled in response to the first electrical state and disabled in response to the second electrical state. The second electrical state will prevent the flow control apparatus from operating while causing an alarm to be activated when operation of the flow control apparatus is attempted.

As an example of the present invention there is presented a flow control apparatus which is arranged to receive a corresponding administration feeding set. The switching component is responsive to the proper mounting of the administration feeding set and is provided with a control means responsive to the switching component for permitting operation of the flow control apparatus only when the switching component detects proper mounting of the administration feeding set. Preferably, the switching component is operatively associated with a sensor arrangement comprising a Hall-effect sensor and magnet reconfigured to be back-biased to enable detection of a magnetically-susceptible metallic component on the administration feeding set by the switching component when the feeding set is properly mounted to the flow control apparatus.

Additional objects, advantages and novel features of the invention will be set forth in the description which follows, and will become apparent to those skilled in the art upon examination of the following more detailed description and drawings in which like elements of the invention are similarly numbered throughout.

BRIEF DESCRIPTION OF THE DRAWINGS

FIG. 1 is a perspective view of the enteral feeding system according to the present invention;

FIG. 2 is a perspective view of the enteral feeding system illustrating the interior of the flow control apparatus according to the present invention;

FIG. 3A is a perspective view illustrating the internal arrangement of the safety interlock system according to the present invention;

FIGS. 3B-3D are perspective views illustrating the improper placement of the safety interlock system according to the present invention;

FIG. 4 is a schematic diagram illustrating the electrical connection of a switching component according to the present invention;

FIG. 4a is a flow chart illustrating the flow logic of the microcontroller according to the present invention;

FIG. 5 is a top view of a mounting member according to the present invention;

FIG. 6 is a cross-sectional view of the mounting member taken along lines 5-5 of FIG. 4 according to the present invention;

FIG. 7 is a cross-sectional view of a safety interlock system taken along lines 6-6 of FIG. 1 according to the present invention;

FIG. 8 is plan view of a magnetically-susceptible metallic component according to the present invention;

FIG. 9 is a cross-sectional view of the magnetically-susceptible metallic component taken along lines 8-8 of FIG. 7 according to the present invention;

FIG. 10 is a simplified block diagram showing a preferred embodiment of the safety interlock system using a back biased sensor arrangement according to the present invention;

FIG. 10A is a simplified block diagram of an alternative arrangement shown in FIG. 10 of the safety interlock system according to the present invention.

FIG. 11 is a simplified block diagram showing an alternative embodiment of the safety interlock system using a resonant coil arrangement according to the present invention;

FIG. 11A is a schematic diagram showing of the alternative embodiment shown in FIG. 11 of the safety interlock system according to the present invention;

FIG. 12 is a schematic diagram showing another alternative embodiment of the safety interlock system using a balanced coil arrangement according to the present invention; and

FIG. 13 is a schematic diagram showing another alternative embodiment of the safety interlock system using a differential active coil arrangement according to the present invention.

DETAILED DESCRIPTION OF THE INVENTION

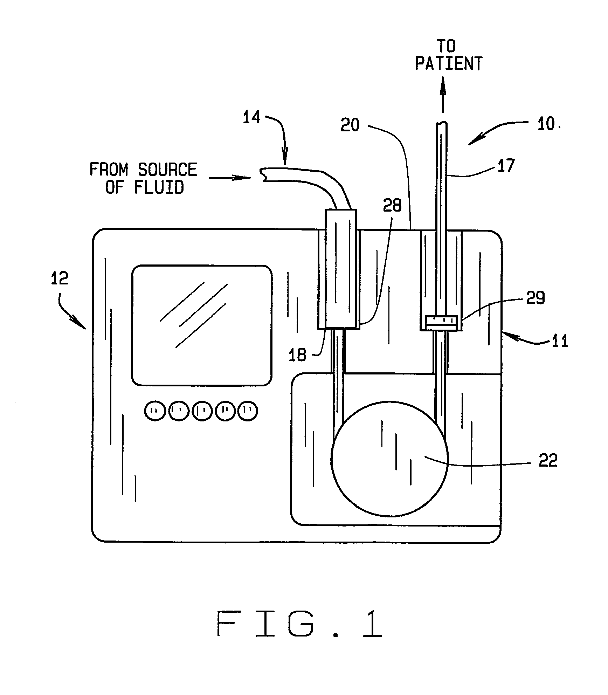

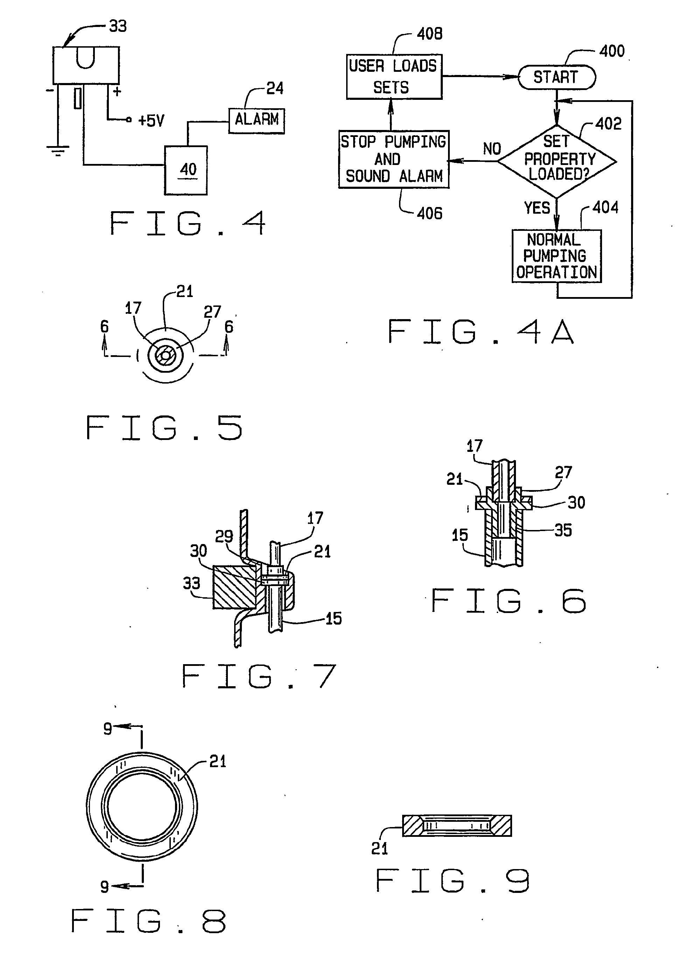

Referring to the drawings, the preferred embodiment of the safety interlock system is illustrated and generally indicated as 11 in FIG. 1. Safety interlock system 11 is preferably used with a flow control apparatus 12, such as a peristaltic pump or other suitable infusion device, of an enteral feeding system 10 for providing fluid to a patient's gastrointestinal tract (not shown) at a controlled rate of delivery. An administration feeding set 14 is operatively engaged with a flow control apparatus 12 for transporting fluid through an inlet tube 16 connected to a source of fluid (not shown) at one end and a pump tube 15 at the opposite end (FIGS. 6 and 7). Administration feeding set 14 further includes a support member 18 attached between the inlet tube 16 and the pump tube 15 for direct engagement of the feeding set 14 to the flow control apparatus 12 as shall be explained in greater detail below. In an alternative embodiment of administration feeding set 14, a drip chamber (not shown) may be substituted for the support member 18 in order to control the flow of fluid through the feeding set 14.

Referring to FIGS. 1, 2 and 3, flow control apparatus 12 comprises a rotor 22 driven by a motor (not shown) that rotates rotor 22 at a predetermined speed at specific time intervals between cycles of rotor 22 to control the rate of delivery of fluid through administration feeding set 14. In addition, delivery of fluid may also be controlled by varying the angle of rotation of rotor 22 on each cycle. A housing 20 encloses the motor and forms recesses 28 and 29 for properly mounting of administration feeding set 14. As shown, pump tube 15 is attached to support member 18 at its inlet end and to a mounting member 30 at its outlet end. When properly engaging the administration feeding set 14 to flow control apparatus 12, support member 18 is seated in recess 28, pump tube 15 is engaged around rotor 22, and mounting member 30 is properly mounted in recess 29.

Referring to FIGS. 1, 5 and 6, mounting member 30 is attached to the outlet end of pump tube 15 by an engaging member 35 on the bottom side of member 30 and a tube receiving member 27 at the top side thereof. As further shown, a circular shaped magnetically-susceptible metallic component 21 is disposed within mounting member 30 which surrounds pump tube 15. An outlet tube 17 is attached to the tube receiving member 27 for allowing fluid flow to the patient from pump tube 15.

In accordance with one aspect of the present invention, safety interlock system 11 provides a means for assuring the proper placement of administration feeding set 14 on flow control apparatus 12, and in particular with the proper placement of the pump tube 15 around the rotor 22 to form a peristaltic pump which provides accurate and controlled delivery rate of fluid through feeding set 14.

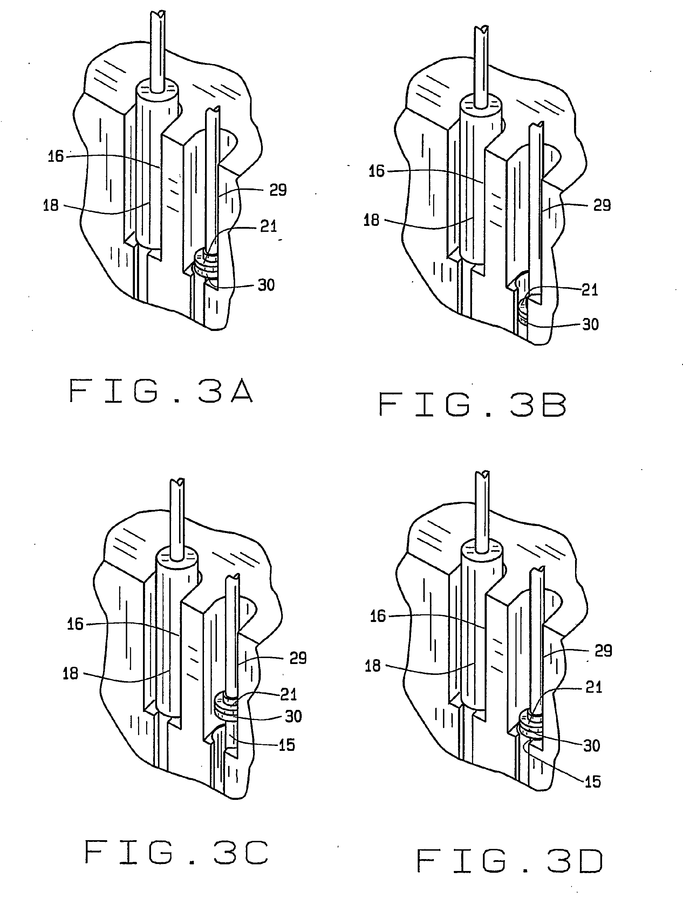

As illustrated in FIGS. 1 and 3A, support member 18 is received in recess 28 and mounting member 30 is received in recess 29. When properly mounted, pump tube 15, which is typically made from silicone tubing, is tightly stretched around rotor 22 so that the points of the rotor 22 contact on pump tube 15 and progressively close the conduit 23 thereof during operation. Referring to FIG, 3A, there is shown the proper placement of the administration feeding set 14 on flow control apparatus 12 such that the support member 18 is properly seated in recess 28 and mounting member 30 is mounted into recess 29.

A person of ordinary skill in the art will appreciate that in some cases the mounting member 30 of safety interlock system 11 may be improperly installed by inexperienced personnel so that the mounting member 30 is seated below recess 29 as shown in FIG. 3B. This particular arrangement can be hazardous to a patient because of excess and uncontrolled flow of fluid through the administration feeding set under force of gravity, whereby fluid is delivered at a higher rate than properly specified for the patient. Another improper mounting of the administration feeding set is illustrated in FIG. 3C, wherein mounting member 30 is caught on the lip of recess 29 and not properly seated therein. Finally, another improper mounting is shown in FIG. 3D wherein mounting member 30 is not placed completely back into recess 29, but seats on the outer edge thereof. On the other hand, experience has shown that improper placement of drip chamber 18 itself is unlikely since it must be seated in both recesses 26 and 28 in order to be properly engaged.

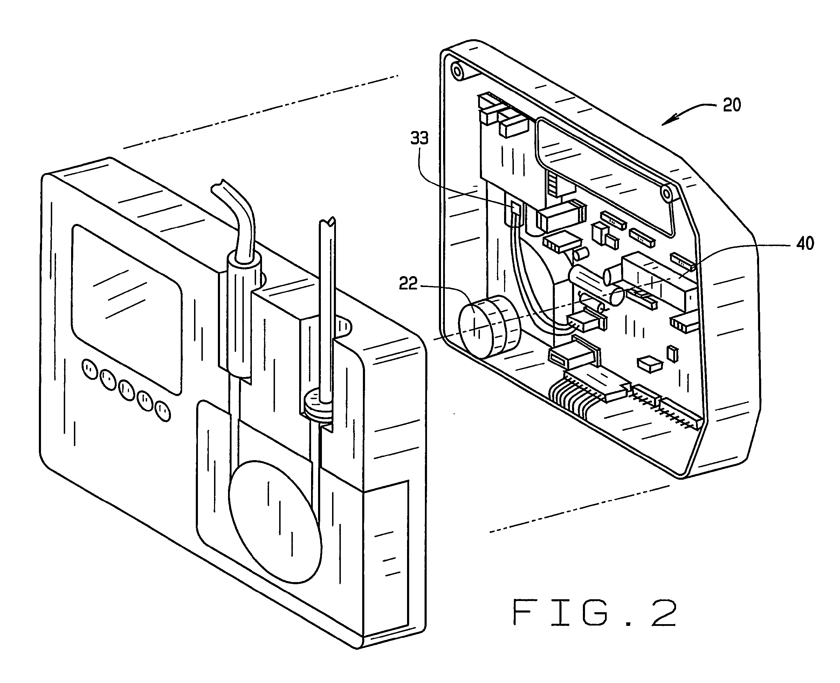

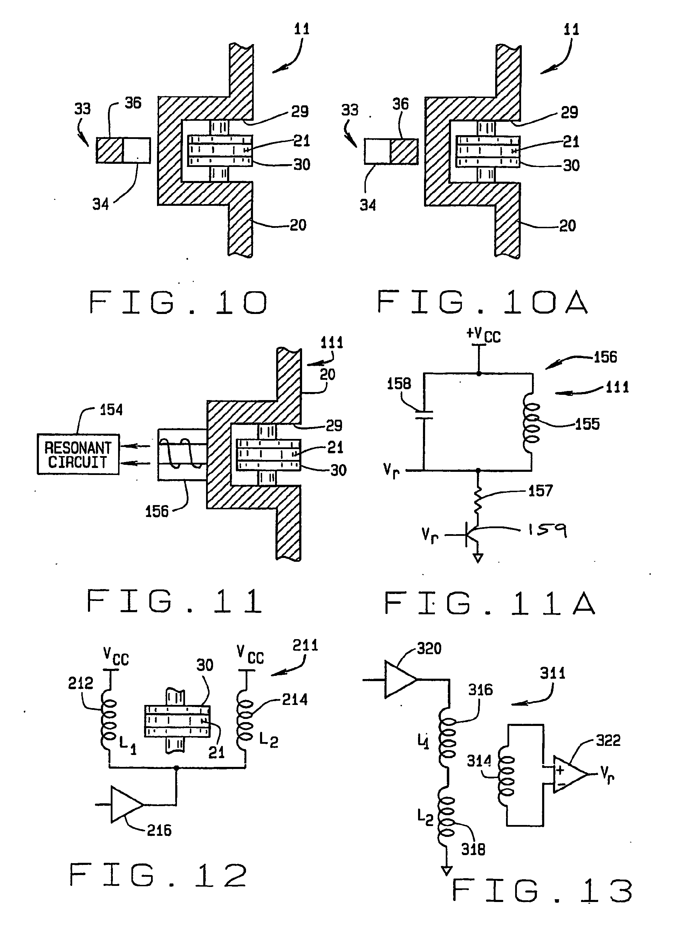

In accordance with a preferred embodiment (FIGS 2, 7 and 10) of the present invention, a sensor arrangement 33 preferably comprising a back-biased sensor 34 is provided inside flow control apparatus 12 for detecting the proper placement of mounting member 30 in recess 29. Referring to FIGS. 5-9, the metallic component 21, preferably a ferrous alloy, is used to detect the proper placement of mounting member 30 in recess 29 which is a toroidal shaped component added to mounting member 30 that surrounds the fluid passage of pump tube 15. Preferably, back-biased sensor 34 is provided within housing 20 and arranged to detect the presence of the metallic component 21 when mounting member 30 is properly seated within recess 29. FIGS. 1 and 2 show the internal arrangement of housing 20 indicating the proper placement of metallic component 21 against the inner wall adjacent recess 29. Back-biased sensor 34 comprises a sensor arrangement 33 including a biasing magnet 36 operatively associated with a Hall-Effect or magneto-resistive sensor 34, as illustrated in FIG. 10. In configuration, sensor 34 is interposed between recess 29 and biasing magnet 36 such that the magnetic flux between the magnet 36 and sensor 34 is changed by the presence of the metallic component 21 when the mounting member 30 is properly seated in recess 29. This change in magnetic flux caused by the metallic component 21 being properly received within recess 29 is sensed by sensor arrangement 33 and a signal is communicated to a microcontroller 40 (FIG. 4), or other control means, which will halt the operation of flow control apparatus 12 and an alarm is sounded as shown in FIG. 4. Therefore, when the mounting member 30 is disengaged from the recess 29, improperly seated therein, or the metallic component 21 is displaced during operation, the metallic component 21 is not sensed by the sensor arrangement 33 and the microcontroller 40 terminates operation of the flow control apparatus 12 and sound an alarm. This operation is illustrated in FIG. 4A wherein after the operation of flow control apparatus 12 is started at step 400, the microcontroller 40 determines whether the administration feeding set 14, and more particularly the mounting member 30, is properly seated in recess 29. If the microcontroller 40 determines that the administration feeding set 14 is properly mounted, then normal operation of the flow control apparatus 12 is permitted at step 404. However, if the microcontroller 40 determines that the administration feeding set 14 is improperly mounted to the recess 29, then operation of the flow control apparatus 12 is terminated and an alarm is sounded at step 406. After operation is terminated, the user must then re-load the administration feeding set 14 to the flow control apparatus at step 408 which allows the microcontroller 40 to start operation at step 400.

In accordance with another aspect of the present invention an alternative embodiment 111 of safety interlock system is contemplated. As shown in FIGS. 11 and 11A, safety interlock system 111 comprises a resonant circuit 154 with a coil arrangement 156 which collectively act as a resonant coil arrangement or tank circuit. In configuration, coil arrangement 156 is disposed along the inner housing 20 of flow control apparatus 12 such that arrangement 156 is directly interposed between the resonant circuit 154 and metallic component 21 when component 21 is properly received within recess 29. As shown in FIG. 11A, resonant circuit 154 comprises a capacitor 158 in parallel with an inductive coil 155 which are both connected to a transistor 159 through a resistor 157 that produces a resonant frequency with coil ar-

rangement 156 of:

$$\tfrac{1}{2}\prod\sqrt{LC}$$

where L is the inductance of inductive coil 155 and C is the capacitance of capacitor 158.

In operation, when metallic component 21 is properly received within recess 29, the presence of component 21 changes the resonant frequency produced by resonant circuit 154. The change in inductance produced in inductive coil 155 in the presence of metallic component 21 changes the resonant frequency to:

$$\tfrac{1}{2}\prod\sqrt{L_1 C}$$

where L1 is the inductance of inductive coil 155 in the presence of metallic component 21. This change in resonant frequency is detected at Vr, the voltage response of the resonant circuit 154, which is then communicated to microcontroller 40.

In accordance with another aspect of the present invention an alternative embodiment 211 of safety interlock system is contemplated. Referring to FIG. 12, safety interlock system 211 comprises a first inductive coil 212 in parallel with a second inductive coil 214 which are both driven by a driver circuit 216 connected to a power source (not shown) which collectively act as a balanced coil arrangement. First and second inductive coils 212 and 214 are wound in opposing directions such that the inductive coils 212 and 214 produce a resonant frequency of:

$$\tfrac{1}{2}\prod\sqrt{(L_1 L_2)}$$

where L1 and L2 are the inductance of the first and second inductive coils 212 and 214, respectively. The above arrangement assumes that the first and second inductive coils 212 and 214 are sufficiently spaced apart as to have negligible mutual interactions.

In configuration, first and second inductive coils 212 and 214 are arranged such that metallic component 21 is placed directly between inductive coils 212 and 214 when properly received within recess 29. Such placement of the metallic component 21 changes the resonant frequency produced between first and second inductive coils 212 and 214 by altering the coupling between the first and second inductive coils 212 and 214 by reducing the sum total of L1 and L2 which can then be detected at voltage Vcc and communicated to microcontroller 40.

In accordance with another aspect of the present invention an alternative embodiment 311 of safety interlock system is contemplated. As illustrated in FIG. 13, safety interlock system 311 comprises a driver circuit 320 which drives a first driver inductive coil 316 in series with a second driver inductive coil 318 that collectively act as a differential active coil arrangement. First and second driver inductive coils 316 and 318 are in close proximity with a sense inductive coil 314 which is operatively associated with an differential amplifier 322. In configuration, safety interlock system 311 is arranged such that metallic component 21 is placed adjacent to driver inductive coil 318. The sense inductive coil 314 is located between the first and second driver inductive coils 316 and 318. When no metallic component 21 is present, there is only a small voltage, Vr, produced at the output of differential amplifier 322. However, in the presence of the metallic component 21 within recess 29 the inductance of second driver inductive coil 318 is decreased relative to first driver inductive coil 316 which unbalances the system 311 and causes the voltage at the output of amplifier 322 to increase. This unbalancing of safety interlock system 311 is sensed at Vr, the response voltage of sense coil 314, which is then communicated to microcontroller 40.

It is contemplated that the safety interlock system 11 can be used with different types of flow control apparatuses 12. For example, flow control apparatuses 12 such as peristaltic, linear peristaltic and rotary peristaltic pumps are felt to fall within the present invention. Further, the fluid administration set 14 can have a drip chamber for providing a visual indication of fluid flow, or an ultrasonic sensor arrangement, such as a piezoelectric transmitter and receiver, may be attached to the flow control apparatus 12 in order to detect fluid flow in lieu of a drip chamber.

**Claims**

1. An enteral feeding system (10) comprising:

   an administration feeding set (14) forming a conduit (23) adapted for fluid flow communication therethrough, the administration feeding set including a component (21), and
   a flow control apparatus (12) operatively associated with said administration feeding set (14) for establishing fluid flow through said conduit (23),
   **characterized in that** the administration feeding set is a magnet-less administration feeding set but wherein said component (21) is magnetically susceptible and that said flow control apparatus (12) further includes a sensor arrangement (33), including a magnetic field source for sensing the presence of said component (21) properly received on the flow control apparatus (12), wherein said component (21) interferes with said magnetic field source and said interference is detectable by said sensor arrangement (33) when said administration feeding set (14) is properly engaged to said flow control apparatus (12).

2. The enteral feeding system (10) according to claim 1, wherein said flow control apparatus (12) further includes a recess (29) for receiving the component (21).

3. The enteral feeding system (10) according to claim 2 wherein said sensing arrangement detects the presence of the component (21) only when the component (21) is properly seated within the recess (29).

4. The enteral feeding system (10) according to claim 3, wherein said administration feeding set (14) further includes a mounting member (30) for mounting said component (21) in said recess (29).

5. The enteral feeding system (10) according to claim 1, wherein said sensor arrangement (33) comprises a magnet (36) operatively associated with a sensor (34).

6. The enteral feeding system (10) according to claim 5, wherein magnetic flux lines present between said magnet (36) and said component (21) change when said component (21) is properly received in said recess (29) which is sensed by said sensor (34).

7. The enteral feeding system (10) according to claim 1, wherein said sensor arrangement (33) comprises a back-biased sensor (36) and magnet (34) wherein said sensor (36) senses the presence of said component (21) when said administration feeding set (14) is properly engaged to said flow control apparatus (12) by the change in magnetic flux lines between said magnet (34) and said component (21).

8. The enteral feeding system (10) according to claim 1, wherein said sensor arrangement (33) comprises a resonant circuit (154) with a coil arrangement (156) which sense the presence of said component (21) when said administration feeding set (14) is properly engaged to said flow control apparatus (12).

9. The enteral feeding system (10) according to claim 8, wherein said resonant circuit (154) and said coil arrangement (156) comprise a capacitor (158) in parallel with an inductive coil (155) which are in series with a transistor (159) through a resistor (157).

10. The enteral feeding system (10) according to claim 1, wherein said sensor arrangement (33) comprises a first inductive coil (212) in parallel with a second inductive coil (214) which sense the presence of said component (21) when said administration feeding set (14) is properly engaged to said flow control apparatus (12).

11. The enteral feeding system (10) according to claim 1, wherein said sensor arrangement (33) comprises a first inductive coil (316) in series with a second inductive coil (318), said first and second inductive coils (316, 318) being in parallel with a third inductive coil (314) which collectively sense the presence of said component (21) when said administration feeding set (14) is properly engaged to said flow control apparatus (12).

12. The enteral feeding system (10) according to claim 11, wherein said third inductive coil (314) is operatively associated with an amplifier (322), said amplifier (322) including a voltage output that increases in the presence of said component (21).

13. The enteral feeding system (10) according to claim 1, wherein said sensor arrangement (33) is operatively associated with a microcontroller (40), said microcontroller (40) permitting operation of said flow control apparatus (12) when said administration feeding set (14) is properly engaged to said flow control apparatus (12) and prevents operation thereof when said administration feeding set (14) in improperly engaged to said flow control apparatus (12).

14. The enteral feeding system (10) according to claim 1, wherein said component (21) is made of a ferrous alloy.

15. The enteral feeding system (10) according to claim 1, wherein the sensor arrangement (33) senses the proper engagement of said component (21) to said flow control apparatus (12) by detecting a change in magnetic flux lines caused by the presence of the component (21) adjacent to the sensing arrangement (33).

**Patentansprüche**

1. Enterales Zuführsystem (10), das Folgendes umfasst: ein Verabreichungszuführset (14), das eine Leitung (23) zur Flüssigkeitsströmungsverbindung durch sie hindurch bildet, wobei das Verabreichungsset eine Komponente (21) aufweist, und ein Strömungskontrollgerät (12), das mit dem Verabreichungszuführset (14) zur Erzeugung einer Flüssigkeitsströmung durch die Leitung (23) in Wirkverbindung steht, **dadurch gekennzeichnet, dass** das Verabreichungszuführset ein magnetloses Verabreichungszuführset ist, aber wobei die Komponente (21) magnetisch empfänglich ist, und dass das Strömungskontrollgerät (12) ferner eine Sensoranordnung (33) mit einer Magnetfeldquelle zum Nachweis der Anwesenheit der richtig auf dem Strömungskontrollgerät (12) aufgenommenen Komponente (21) aufweist, worin die Komponente (21) die Magnetfeldquelle stört und die Störung von der Sensoranordnung (33) nachgewiesen werden kann, wenn das Verabreichungszuführset (14) richtig mit dem Strömungskontrollgerät (12) in Eingriff steht.

2. Enterales Zuführsystem (10) nach Anspruch 1, worin das Strömungskontrollgerät (12) ferner eine Vertiefung (29) zur Aufnahme der Komponente (21) aufweist.

3. Enterales Zuführsystem (10) nach Anspruch 2, worin die Sensoranordnung das Vorhandensein der Komponente (21) nur nachweist, wenn die Komponente (21) richtig in der Vertiefung (29) sitzt.

4. Enterales Zuführsystem (10) nach Anspruch 3, worin das Verabreichungszuführset (14) ferner ein Befestigungselement (30) zur Befestigung der Komponente (21) in der Vertiefung (29) aufweist.

5. Enterales Zuführsystem (10) nach Anspruch 1, worin die Sensoranordnung (33) einen Magneten (36) aufweist, der mit einem Sensor (34) in Wirkverbindung steht.

6. Enterales Zuführsystem (10) nach Anspruch 5, worin magnetische Flusslinien, die zwischen dem Magneten (36) und der Komponente (21) anwesend sind, sich verändern, wenn die Komponente (21) richtig in der Vertiefung (29) aufgenommen ist, was vom Sensor (34) nachgewiesen wird.

7. Enterales Zuführsystem (10) nach Anspruch 1, worin die Sensoranordnung (33) einen rückwärts vorgespannten Sensor (36) und einen Magneten (34) umfasst, worin der Sensor (36) die Anwesenheit der Komponente (21) durch die Veränderung der magnetischen Flusslinien zwischen dem Magneten (34) und der Komponente (21) nachweist, wenn das Verabreichungszuführset (14) richtig mit dem Strömungskontrollgerät (12) in Eingriff steht.

8. Enterales Zuführsystem (10) nach Anspruch 1, worin die Sensoranordnung (33) einen Resonanzkreislauf (154) mit einer Spulenanordnung (156) umfasst, die das Vorhandensein der Komponente (21) nachweisen, wenn das Verabreichungszuführsystem (14) richtig mit dem Strömungskontrollgerät (12) in Eingriff steht.

9. Enterales Zuführsystem (10) nach Anspruch 8, worin der Resonanzkreislauf (154) und die Spulenanordnung (156) einen Kondensator (158) parallel zu einer Induktionsspule (155), die über einen Widerstand (157) mit einem Transistor (159) in Reihe geschaltet sind, umfassen.

10. Enterales Zuführsystem (10) nach Anspruch 1, worin die Sensoranordnung (33) eine erste Induktionsspule (212) parallel zu einer zweiten Induktionsspule (214) umfasst, die das Vorhandensein der Komponente (21) nachweisen, wenn sich das Verabreichungszuführset (14) richtig mit dem Strömungskontrollgerät (12) in Eingriff befindet.

11. Enterales Zuführsystem (10) nach Anspruch 1, worin die Sensoranordnung (33) eine erste Induktionsspule (316) umfasst, die mit einer zweiten Induktionsspule (318) in Reihe geschaltet ist, wobei die erste und zweite Induktionsspule (316, 318) parallel zu einer dritten Induktionsspule (314) angeordnet sind und zusammen mit dieser das Vorhandensein der Komponente (21) nachweisen, wenn sich das Verabreichungszuführset (14) richtig mit dem Strömungskontrollgerät (12) in Eingriff befindet.

12. Enterales Zuführsystem (10) nach Anspruch 11, worin die dritte Induktionsspule (314) mit einem Verstärker (322) in Wirkverbindung steht, wobei der Verstärker (322) einen Spannungsausgang aufweist, der in Anwesenheit der Komponente (21) zunimmt.

13. Enterales Zuführsystem (10) nach Anspruch 1, worin die Sensoranordnung (33) mit einem Mikroregler (40) in Wirkverbindung steht, wobei der Mikroregler (40) den Betrieb des Strömungskontrollgeräts (12) zulässt, wenn sich das Verabreichungszuführset (14) richtig mit dem Strömungskontrollgerät (12) in Eingriff befindet und dessen Betrieb verhindert, wenn sich das Verabreichungszuführset (14) nicht richtig mit dem Strömungskontrollgerät (12) in Eingriff befindet.

14. Enterales Zuführsystem (10) nach Anspruch 1, worin die Komponente (21) aus einer Eisenlegierung besteht.

15. Enterales Zuführsystem (10) nach Anspruch 1, worin die Sensoranordnung (33) den richtigen Eingriff der Komponente (21) mit dem Strömungskontrollgerät (12) nachweist, indem sie eine Veränderung der magnetischen Flusslinien nachweist, die durch die Anwesenheit der Komponente (21) neben der Sensoranordnung (33) hervorgerufen wird.

**Revendications**

1. Système d'alimentation entérale (10) comprenant :

   un ensemble d'alimentation d'administration (14) formant un conduit (23) prévu pour assurer à travers lui une communication d'écoulement fluidique, l'ensemble d'alimentation d'administration comportant un composant (21), et un appareil de régulation de l'écoulement (12) associé fonctionnellement audit ensemble d'alimentation d'administration (14) pour établir un écoulement fluidique à travers ledit conduit (23), **caractérisé en ce que** l'ensemble d'alimentation d'administration est un ensemble d'alimentation d'administration sans aimant mais dans lequel ledit composant (21) est sensible magnétiquement, et **en ce que** ledit appareil de régulation de l'écoulement (12) comporte en outre un agencement de capteur (33), comportant une source de champ magnétique pour détecter la présence dudit composant (21) correctement reçu sur l'appareil de régulation de l'écoulement (12), ledit composant (21) interférant avec ladite source de champ magnétique et ladite interférence pouvant être détectée par ledit agencement de capteur (33) lorsque ledit ensemble d'alimentation d'administration (14) est correctement engagé avec ledit appareil de régulation de l'écoulement (12).

2.  Système d'alimentation entérale (10) selon la revendication 1, dans lequel ledit appareil de régulation de l'écoulement (12) comporte en outre un retrait (29) pour recevoir le composant (21).

3.  Système d'alimentation entérale (10) selon la revendication 2, dans lequel ledit agencement de capteur détecte la présence du composant (21) uniquement lorsque le composant (21) est correctement reçu dans le retrait (29).

4.  Système d'alimentation entérale (10) selon la revendication 3, dans lequel ledit ensemble d'alimentation d'administration (14) comporte en outre un organe de montage (30) pour monter ledit composant (21) dans ledit retrait (29).

5.  Système d'alimentation entérale (10) selon la revendication 1, dans lequel ledit agencement de capteur (33) comprend un aimant (36) associé fonctionnellement à un capteur (34).

6.  Système d'alimentation entérale (10) selon la revendication 5, dans lequel des lignes de flux magnétique présentes entre ledit aimant (36) et ledit composant (21) changent lorsque ledit composant (21) est reçu correctement dans ledit retrait (29), ce qui est détecté par ledit capteur (34).

7.  Système d'alimentation entérale (10) selon la revendication 1, dans lequel ledit agencement de capteur (33) comprend un capteur polarisé en inverse (36) et un aimant (34), ledit capteur (36) détectant la présence dudit composant (21), lorsque ledit ensemble d'alimentation d'administration (14) est correctement engagé avec ledit appareil de régulation de l'écoulement (12), par le changement des lignes de flux magnétique entre ledit aimant (34) et ledit composant (21).

8.  Système d'alimentation entérale (10) selon la revendication 1, dans lequel ledit agencement de capteur (33) comprend un circuit de résonance (154) avec un agencement de bobine (156) qui détectent la présence dudit composant (21) lorsque ledit ensemble d'alimentation d'administration (14) est correctement engagé avec ledit appareil de régulation de l'écoulement (12).

9.  Système d'alimentation entérale (10) selon la revendication 8, dans lequel ledit circuit de résonance (154) et ledit agencement de bobine (156) comprennent un condensateur (158) monté en parallèle avec une bobine inductive (155), lesquels sont montés en série avec un transistor (159) par le biais d'une résistance (157).

10. Système d'alimentation entérale (10) selon la revendication 1, dans lequel ledit agencement de capteur (33) comprend une première bobine inductive (212) montée en parallèle avec une deuxième bobine inductive (214), lesquelles détectent la présence dudit composant (21) lorsque ledit ensemble d'alimentation d'administration (14) est correctement engagé avec ledit appareil de régulation de l'écoulement (12).

11. Système d'alimentation entérale (10) selon la revendication 1, dans lequel ledit agencement de capteur (33) comprend une première bobine inductive (316) montée en série avec une deuxième bobine inductive (318), lesdites première et deuxième bobines inductives (316, 318) étant montées en parallèle avec une troisième bobine inductive (314), lesquelles détectent collectivement la présence dudit composant (21) lorsque ledit ensemble d'alimentation d'administration (14) est correctement engagé avec ledit appareil de régulation de l'écoulement (12).

12. Système d'alimentation entérale (10) selon la revendication 11, dans lequel ladite troisième bobine inductive (314) est associée fonctionnellement à un amplificateur (322), ledit amplificateur (322) comportant une sortie de tension qui augmente en présence dudit composant (21).

13. Système d'alimentation entérale (10) selon la revendication 1, dans lequel ledit agencement de capteur (33) est associé fonctionnellement à un microcontrôleur (40), ledit microcontrôleur (40) permettant le fonctionnement dudit appareil de régulation de l'écoulement (12) lorsque ledit ensemble d'alimentation d'administration (14) est correctement engagé avec ledit appareil de régulation de l'écoulement (12) et empêche son fonctionnement lorsque ledit ensemble d'alimentation d'administration (14) n'est pas correctement engagé avec ledit appareil de régulation de l'écoulement (12).

14. Système d'alimentation entérale (10) selon la revendication 1, dans lequel ledit composant (21) est fabriqué en un alliage ferreux.

15. Système d'alimentation entérale (10) selon la revendication 1, dans lequel l'agencement de capteur (33) détecte l'engagement correct dudit composant (21) avec ledit appareil de commande de l'écoulement (12) en détectant un changement des lignes de flux magnétique causé par la présence du composant (21) à côté de l'agencement de capteur (33).

TO
PATIENT

FROM SOURCE
OF FLUID

FIG.1

FIG.2

FIG.3A

FIG.3B

FIG.3C

FIG.3D

FIG.4

FIG.4A

FIG.5

FIG.6

FIG.7

FIG.8

FIG.9

FIG. 10

FIG. 10A

FIG. 11

FIG. 11A

FIG. 12

FIG. 13

**EP 1 542 742 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4913703 A
- US 5201711 A
- US 6017326 A, Pasqualucci